# NEUE EUROPÄISCHE PATENTSCHRIFT

(11) **EP 1 697 006 B2**
(45) Veröffentlichungstag und Bekanntmachung des Hinweises auf die Entscheidung über den Einspruch: **23.02.2011**
(45) Hinweis auf die Patenterteilung: 26.12.2007
(21) Anmeldenummer: 04797806.9
(22) Anmeldetag: 11.11.2004
(51) Int. Cl.: A61Q 19/00, A61K 8/64

(54) **VERWENDUNG VON PEPTIDEN ZUM SCHUTZ DER HAUT VOR HAARBEHANDLUNGSMITTELN**
USE OF PEPTIDES TO PROTECT SKIN FROM HAIR TREATMENT AGENTS
UTILISATION DE PEPTIDES POUR PROTEGER LA PEAU CONTRE DES AGENTS DE TRAITEMENT CAPILLAIRE

(30) Priorität: 28.11.2003 DE 10355743
(43) Veröffentlichungstag der Anmeldung: 06.09.2006
(73) Patentinhaber: Wella Aktiengesellschaft, 64274 Darmstadt (DE)
(72) Erfinder: ROTHE, Helga, 64293 Darmstadt (DE); DETAPPE, Veronique, CH-1700 Fribourg (CH); NOSER, Friedrich, CH-1724 Bonnefontaine (CH)
(74) Vertreter: Marollé, Patrick Pierre Pascal
(86) Internationale Anmeldenummer: PCT/EP2004/012768
(87) Internationale Veröffentlichungsnummer: WO 2005/060929

(56) Entgegenhaltungen:
- EP-A2- 0 457 565
- WO-A1-01/43704
- WO-A1-03/030860
- WO-A2-03/077856
- DE-A1- 19 941 819
- US-A- 4 592 908
- US-A- 5 437 860
- PATENT ABSTRACTS OF JAPAN Bd. 1997, Nr. 04, 30. April 1997 (1997-04-30) & JP 08 333233 A (KAO CORP), 17. Dezember 1996 (1996-12-17)
- 'Das Friseurbuch,Kenntnisse und Fertigkeiten', 1990, HANDWERK UND TECHNNIK, HAMBURG, ISBN 358203925 Artikel MÖLLER ,SCHOENEBERG
- 'Körperpflege,Theorie und Praxis für Friseure und Kosmetiker', Bd. 2, 1984, DÜMMLER, BONN, ISBN 3427556417

## Beschreibung

Die vorliegende Erfindung betrifft die nicht medizinische Verwendung von kurzkettigen Peptiden mit einer Kettenlänge zwischen 2 und 30 Aminosäuren zum Schutz der Haut vor Haarbehandlungsmitteln, insbesondere zum Schutz der Kopfhaut vor Farbstoffen oder Färbemitteln, die bekannterweise zum

Anfärben von Haaren Verwendung finden.

Bei der Behandlung von Haaren mit kosmetischen Mitteln ist auch bei sorgfältigster Arbeitsweise ein Hautkontakt mit dem entsprechenden Produkt nicht immer zu vermeiden. Ein solcher Hautkontakt ist bei der Verwendung von Haarfärbemitteln von ganz besonderem Nachteil. Insbesondere an den direkt an den Haaransatz angrenzenden sichtbaren Hautpartien wirken diese Hautanfärbungen störend und sollten beseitigt oder von vorne herein verhindert werden.

Um diesen Nachteil zu verhindern ist es bekannt, verschiedene Emulsionen als Hautschutz vor Haarbehandlungsmitteln einzusetzen. Die erwünschte Wirkung tritt hierbei jedoch nur unzureichend ein, insbesondere bei der Verwendung von Haarfärbemitteln.
Andere Mittel wie zum Beispiel Vaseline müssen nach der Haarbehandlung wieder entfernt werden und haben ausserdem den Nachteil, dass sie aufgrund ihrer Unlöslichkeit in Wasser nur sehr schwer und gegebenenfalls unter Einsatz hautreizender Lösungsmittel wieder von der Haut und dem Haaransatz zu entfernen sind.

Es bestand daher die Aufgabe, Zubereitungen zur nicht medizinische Verwendung für die Verhinderung des Hautkontakts von kosmetischen Mitteln, insbesondere Haarfärbemitteln, bereit zu stellen, die die Nachteile herkömmlicher Zubereitungen nicht aufweisen.

Es wurde nun überraschenderweise gefunden, dass die nicht medizinische Verwendung einer Zubereitung mit einem Gehalt an kurzkettigen Peptiden zum Schutz der Haut vor einem unerwünschten in Kontakt treten mit Mitteln zur kosmetischen Behandlung der Haare, insbesondere zum Schutz der Haut vor einem unerwünschten Anfärben der Haut beim Färben oder Tönen von Haaren, die gestellte Aufgabe hervorragend löst.

Der Schutz der Haut vor einem unerwünschten in Kontakt treten mit Mitteln zur kosmetischen Behandlung der Haare kann solche Mittel umfassen, die das Haar färben, tönen, verformen, härten, konditionieren, weichmachen, reparieren oder stylen können. Dem Fachmann sind sowohl diese Wirkungen als auch die diese Wirkungen hervorrufenden Haarbehandlungsmittel bekannt.

Folglich betrifft die vorliegende Erfindung die nicht medizinische Verwendung einer Zubereitung mit einem Gehalt an kurzkettigen Peptiden zum Schutz der Haut vor Haarbehandlungsmitteln.
Insbesondere betrifft die vorliegende Erfindung die nicht medizinische Verwendung einer Zubereitung mit einem Gehalt an kurzkettigen Peptiden zum Schutz der Haut vor Haarbehandlungsmittel, die Mittel umfassen, die das Haar färben, tönen, verformen, härten, konditionieren, weichmachen, reparieren oder stylen können.
Ganz besonders betrifft die vorliegende Erfindung die nicht medizinische Verwendung einer Zubereitung mit einem Gehalt an kurzkettigen Peptiden zum Schutz der Haut vor solchen Haarbehandlungsmitteln, die das Haar färben oder tönen können und somit ein unerwünschtes Anfärben der Haut, insbesondere der Kopfhaut, zu verhindern oder zu vermindern vermögen.

Dadurch, dass die erfindungsgemäße nicht medizinische Verwendung der Peptide den Hautkontakt mit Haarbehandlungsmitteln vermeidet oder zumindest vermindert, können unerwünschte Erscheinungen der Haut vermieden oder gemildert werden. Diese unerwünschten Erscheinungen können insbesondere Anfärbungen umfassen.

Insbesondere beim Färben von Haaren stellt das unerwünschte Mitanfärben der betreffenden Kopfhaut ein besonderes Problem für den Frisör aber auch für den Privatanwender dar.

Besonders bevorzugt ist daher die erfindungsgemäße nicht medizinische Verwendung zum Schutz der Haut vor einem unerwünschten Anfärben der Haut beim Färben oder Tönen von Haaren.

Die erfindungsgemäß zu verwendenden kurzkettigen Peptide weisen eine Kettenlänge zwischen 2 und 30 Aminosäuren, vorzugsweise zwischen 6 und 15 Aminosäuren, insbesondere zwischen 6 und 12 Aminosäuren auf.

Im Sinne der vorliegenden Erfindung werden solche Peptide verstanden, deren Monomere aus Aminosäuren oder Aminosäurederivaten oder Gemischen davon bestehen, welche üblicherweise durch Säure-Amid-Bindung miteinander verbunden sind. Sie können synthetischen (über technische Totalsynthese hergestellt), semisynthetischen (über Teilsynthese und natürlichen Quellen) oder natürlichen Ursprungs sein, wobei auch gentechnische bzw. mikrobiologische Herstellungsverfahren (zum Beispiel über die bekannten Methoden der DNA-Rekombination), mitumschlossen sind.

Als Peptide können auch solche verwendet werden, deren Aminosäuren Heteroatome oder Heteroatomgruppierungen enthalten können. Als beispielsweise geeignete Aminosäurederivate können alpha-Aminocarbonsäuren mit mindestens drei Kohlenstoffatomen genannt werden, wobei die Heteroatome oder Heteroatomgruppen, wie beispielsweise eine freie Amino-, Hydroxy-, Sulfanyl- oder Carboxylgruppe, bevorzugt an das endständige Kohlenstoffatom gebunden sein können.
Von der Erfindung mitumfasst werden daher auch solche Peptide, die Aminosäuren-Derivate enthalten, welche eine freie Amino-, Hydroxy- oder Sulfanylgruppe enthalten.

Als ganz besonders bevorzugt sind kurzkettige Peptide, die vorzugsweise als 2-mer bis 30-mer vorliegen, insbesondere zwischen 3-mer und 15-mer, ganz besonders als 6-mer bis 12-mer. Beispielsweise kommen Peptide mit folgender Aminsäure-Sequenz (Angabe der Aminosäuren gemäß bekanntem Einbuchstaben-Code) in Betracht:
AAVIQL, ADESKHVWSQT, AFTQGLK, AGTFSTPRKKFKK, AGTVLIEDNNFTNE, ATCESRWT, ATPSILQTPKTT, AVLTEEDSD, DDEENQSLTTKKES, DDENDSYTDHENI, DDTDEIEND, DEENSQT, DEGESTQSVKTPRKK, DELHSA, DENTSENQSE, DENVEDDE, DNEVADN, DYTQMPISWKRK, EDEETEQSLPKKEED, EDHWNDPRSAV, EDNRTPSTAI, EDNTQVIPRKSLTWS, EDSYTQSLPKKTS, EDTSTENKNTNDEE, EKHSYTNLSPR, EKSTANPSQD, ELGQNS, ENDTHMENS, ENSADNDEL, ESEDDMVNTDEE, GAYNYE, GNTRKVEVR, IFTAYQSPRKSTI, ISLTQPKRFW, IVRKSATNSLPKKV, KKETQFKRSTKQSLS, KKFSQLLK, KKRKKKTMIKSK, KKRSLIKKSRPKS, KKRSTSTQLVKRRT, KKRTRLK, KKTRSTLQRKIRK, KRAKRR, KRQSIHSA, KRSKRTKSPKIS, KRWTGCALRKR, LENQEI, LITASFTQSLPRKSG, MAFMTQSVHVT, MAVENDES, MEDMEHSENTEIT, MFSTQTLKR, MGHVQSL, MGTWTQISLPRK, MITQLIPRMS, MLSQTI, MQTISPTARE, MQTSSYIALTMSM, MSTAVLA, NDEHDEHKRVKT, NDSQLDKT, NEDDEFSSSPRKKTS, NEIDEG, NEMVLTQSHNEDE, NEYILDQTLED, NKASIEEDNDPNIRS, NMCTQNLLRKTMSE, NNDECWSAT, NNSPSEETEA, NVRKKLK, RAKRITKFTQSIPKK, RGKKLHRTV, RIKRRSYSTS, RISKKRTYST, RKKSKAVKKI, RKSRKLIYHKMKK, RKVSQLT, RRQSLLTKKAR, RSTIRTHQLKKR, RVHYKK, SAKISKKRSSKPSAV, SATLAHI, SMMSTLYSWSEDMT, SSVTQSLGVIHFYS, STASDHSS, STAVRRSL, SVGLITQSSLPKKSV, TGTSLQHYQSSL, TIAVYTPRKS, TKKRKITQSPEERK, TTQSIKTI, TWSAVHSPQST, VASTSTQSLPTSWS, VGTQSI, VKKRSRSKKKL, VQSAWCTSAD, VSIEDNTEA, VSMENQSA, VSQLSTSQLLTS, VTSLRRA.

Das Auffinden und Selektieren eines für den Zweck der vorliegenden Erfindung geeigneten Peptids kann nach an sich bekannten Methoden erfolgen und ist dem Fachmann bekannt. Ein solches Verfahren kann *in vivo* oder *in vitro* ausgeführt werden und beruht auf einem einfachen vergleichenden Ausprobieren. Beispielsweise eignen sich hierzu Präparate aus Schweinehaut, deren Oberfläche mit einer ein erfindungsgemäßes Peptid enthaltende Zusammensetzung und als Kontrolle kein Peptid enthaltende Zusammensetzung behandelt werden. Diese Hautpräparate werden anschließend mit einem ausgewählten kosmetischen Mittel, beispielsweise einem Haarfärbemittel, in Kontakt gebracht, welches nach einer geeigneten Einwirkzeit wieder abgewaschen wird. Hiernach kann die Menge des auf den Hautpräparaten verbliebenen kosmetische Mittels, beispielsweise ein Farbstoff, quantitativ bestimmt werden.

Bevorzugt werden solche Peptide umfaßt, die in wässriger Lösung im pH-Bereich zwischen pH 4,0 und pH 8,5, vorzugsweise pH 5,0 bis 6,0 eine spezifische Bindung an der Hautoberfläche entfalten.

Die für die vorliegende Erfindung geeigneten Peptide können nach an sich bekannten Methoden hergestellt oder kommerziell erhalten werden, beispielsweise von ORPEGEN Pharma, Heidelberg.
So kann über Routineverfahren ein Peptid der gewünschten Kettenlänge synthetisiert werden, beispielsweise nach der allgemein bekannten Merrifield-Technik.

Desweiteren lassen sich geeignete Peptide identifizieren über das Screenen von Phage-Peptid-Banken (auch als "Phage-Display" bekannt, beispielsweise gemäß Devlin JJ et al. , Science 249, 404 - 406, 1990) und deren mögliche weitere Optimierung durch die Methode des "Cosmix-Plexing" gemäß WO98/33901.

Für die Auswahl der für die Synthese des Peptids geeigneten Aminosäuren kommen im Prinzip alle bekannten Aminosäuren in Betracht, insbesondere Alanin, Arginin, Asparagin, Asparaginsäure, Cystein, Glutamin, Glutaminsäure, Glycin, Histidin, Isoleucin, Leucin, Lysin, Methionin, Phenylalanin,Prolin, Serin, Threonin, Tryptophan, Tyrosin, Valin.

Bei den Haarbehandlungsmitteln, deren Kontakt mit der erfindungsgemäßen Verwendung der Peptide vermieden oder vermindert werden soll, kann es sich um färbende bzw. farbgebende, konditionierende, verformende, härtende, weichmachende, stylende, reparierende und/oder rekonstituierende Haarbehandlungsmittel handeln.

Unter diesen Haarbehandlungsmittel können beispielsweise kationische Polymere, kationische Tenside, Amidoamine, Betainester, Esterquats, Silikonpolyole, synthetische Polymere wie zum Beispiel Acryl-Polymere, Farbstoffe bzw. farbgebende Haarbehandlungsmittel umfassend oxidative, nicht-oxidative, direktziehende, natürliche, synthetische und semisynthetische Farben, wobei unter den direktziehenden Farben Nitrofarbstoffe, Azofarbstoffe, Chinonfarbstoffe, Triphenylmethanfarbstoffe, saure und basische Farbstoffe, zu nennen wären, verstanden werden.
Desweiteren umfasst der Begriff Haarbehandlungsmittel auch sogenannte Reaktivfarbstoffe, die eine Triazinyl-, Sulfatoethylsulfonyl- oder Vinylsulfonylgruppe enthalten wie beispielsweise Reactive Blue 2, Reactive Blue 19, Reactive Red 2, Reactive Orange 16, Reactive Black 5 und Reactive Yellow 2.
Aber auch vor Farbstoffvorstufen soll die Haut erfindungsgemäß geschützt werden. Farbstoffvorstufen sind beispielsweise Halogennitrobenzol-Derivate, die mit Verbindungen mit freien Amino- oder Hydroxygruppen zu Nitrofarbstoffen umgesetzt werden können. Als Beispiele können hier 4-Fluor-3-nitroanilin, 5-Fluor-2-nitroanilin, 1-Chlor-2,4-dinitrobenzol und 1-Fluor-2,4-dinitrobenzol genannt werden.

Als blaue Nitrofarbstoffe können beispielsweise genannt werden:
1,4-Bis[(2'-hydroxyethyl)amino]-2-nitrobenzol
1-(2'-Hydroxyethyl)amino-2-nitro-4-bis-(2"-hydroxyethyl)amino-benzol (HC Blue No. 2),
1-Amino-3-methyl-4-(2'-hydroxyethyl)amino-6-nitro-benzol (HC Violet No. 1),
4,N-Ethyl,N-(2"-hydroxyethyl)amino-1-(2"-hydroxyethyl)amino-2-nitro-benzol-hydrochlorid (HC Blue No. 12),
4-Bis-(2'-hydroxyethyl)amino-1-(2"-methoxyethyl)amino-2-nitrobenzol HC Blue No. 11),
1-(2',3'-Dihydroxypropyl)amino-2-nitro-4-[N-methyl-(2"-hydroxyethyl)-amino]-benzol-Hydrochlorid (HC Blue No. 10),
1-[(2',3'-Dihydroxypropyl)amino]-2-nitro-4-[N-ethyl-2"-(hydroxyethyl)-amino]-benzol-Hydrochlorid (HC Blue No. 9),
1-(3'-Hydroxypropylamino)-2-nitro-4-bis-(2"-hydroxyethylamino)-benzol (HC Violet No. 2),
4,N-Methyl,N-(2',3'-dihydroxypropyl)amino-1-methylamino-2-nitro-benzol-Hydrochlorid (HC Blue No. 6),
4'Amino-2'-nitro-2"-carboxy-4"-dimethylamino-diphenylamin (HC Blue No. 13),

Als rote Nitrofarbstoffe können beispielsweise genannt werden:
1-Amino-4-(2'-hydroxyethyl)-amino-2-nitrobenzol (HC Red No. 7),
1-Hydroxy-2-amino-4,6-dinitro-benzol,
4-Amino-2-nitro-diphenylamin (HC Red No. 1) ,
1-Amino-2-nitro-4-bis-(2'-hydroxyethyl)amino-benzol-Hydrochlorid (HC Red No. 13),
1-Amino-2-nitro-4-(2'-hydroxyethyl)amino-5-chlorbenzol,
1-(2'-Hydroxyethyl)amino-2-nitro-4-amino-benzol (HC Red No. 3),
1-Hydroxy-3-nitro-4-amino-benzol,
1-Hydroxy-3-nitro-4-(2'-hydroxyethylamino)benzol
1-(2'-Aminoethyl)amino-2-nitro-4-(2'-hydroxyethoxy)-benzol (HC Orange No. 2),
3-Nitro-4-(2'-hydroxyethyl)amino-phenylglycerinether (HC Orange No. 3) ,
1-Amino-5-chlor-4-(2',3'-dihydroxypropyl)amino-2-nitro-benzol (HC Red No. 10),
1,4-Bis-[(2',3'-dihydroxypropyl)amino]-5-chlor-2-nitro-benzol (HC Red No. 11),
1-Hydroxy-2-(2'hydroxyethyl)amino-4,6-dinitro-benzol,
3-Nitro-4-ethylamino-benzoesäure,
4-Amino-2-nitro-diphenylamino-2-carbonsäure,
2-Chlor-6-ethylamino-4-nitrophenol,
2-Amino-6-chlor-4-nitrophenol,
1-Hydroxy-3-nitro-4-(3'-hydroxypropylamino)-benzol,
2,5-Diamino-6-nitropyridin,
1,2,3,4-Tetrahydro-6-nitrochinoxalin,
7-Amino-3,4-dihydro-6-nitro-2H-1,4-benzoxazin (HC Red 14).

Als gelbe Nitrofarbstoffe können beispielsweise genannt werden:
1-Amino-2-(2'-hydroxyethyl)amino-5-nitro-benzol (HC Yellow No. 5),
1-(2'-Hydroxyethoxy)-2-(2"-hydroxyethyl)amino-5-nitro-benzol (HC Yellow No. 4),
1-(2'-Hydroxyethyl)amino-2-nitro-benzol (HC Yellow No. 2),
1-Methoxy-2-(2'-hydroxyethyl)amino-5-nitro-benzol,
1-Hydroxy-2-amino-3-nitro-benzol,
1-Amino-2-methyl-6-nitro-benzol,
1-(2'Hydroxyethyl)-oxy-3-methylamino-4-nitro-benzol,
1-Methylamino-2-nitro-5-(2',3'-dihydroxypropyl)-oxybenzol,
1-(2'-Hydroxyethyl)amino-2-hydroxy-4-nitro-benzol (HC Yellow No. 11),
1-Methoxy-3-(2'-aminoethyl)-amino-4-nitro-benzol-Hydrochlorid (HC Yellow No.9),
1-(2'-Ureidoethyl)amino-4-nitro-benzol,
4-(2',3'-Dihydroxypropyl)amino-3-nitro-trifluormethyl-benzol (HC Yellow No. 6),
2,4-Bis-[N-(2'-hydroxyethyl)aminol-5-chlor-nitrobenzol (HC Yellow No. 10),
4-(2'-Hydroxyethyl)amino-3-nitro-methylbenzol,
4-(2'-Hydroxyethyl)amino-3-nitro-chlorbenzol (HC Yellow No. 12),
4-(2'-Hydroxyethyl)amino-3-nitro-trifluormethyl-benzol (HC Yellow No. 13),
4-(2'-Hydroxyethyl)amino-3-nitro-benzonitril (HC Yellow No. 14),
4-(2'-Hydroxyethyl)amino-3-nitro-benzamid (HC Yellow No. 15).

Als Azofarbstoffe können beispielsweise genannt werden:
1-(4'-Nitrophenylazo)-2-methyl-4-bis-(2'-hydroxyethyl)amino-benzol,
1-(3'-Nitro-4-amino)-phenylazo-2-hydroxy-7-trimethyl-ammoniumchlorid-naphthalin,
1-(2'Hydroxy-4'sulfo-6'nitro)-naphthylazo-2-hydroxynaphthalin CI 15700,
1-(4'-Aminophenylazo)-2-methyl-4-bis-[(2'-hydroxyethyl)-amino]-benzol,
5-(4'-Dimethylaminophenylazo)-1,4-dimethyl-triazoniumchlorid,
1-(2'-Methoxyphenylazo)-2-hydroxy-7-trimethylammonium-naphthalinchorid,
1-(4'-Aminophenylazo)-2-hydroxy-7-trimethylammonium-naphthalin,
4-(3'-Trimethylammoniumphenylazo)-N-phenyl-3-methyl-pyrazolon (5),
4-Hydroxy-3-[(4'-sulfo-1'-naphthyl)azo]-1-naphthalinsulfonsäure,
1-(4'-Sulfophenylazo)-2-hydroxynaphthalin,
1-(4'-Sulfonphenylazo)-2-hydroxy-6-sulfo-naphthalin CI 15985,
4-Amino-[4'-bis-(2"-hydroxyethyl)amino]-azobenzol,
4-Amino-[4'-bis-(2"-hydroxyethyl)amino]-2'-methyl-azobenzol,
3-(2',6'-Diaminopyridyl-3'-azo)-pyridin,
7-Phenylazo-1-amino-3,6-disulfo-8-hydroxy-naphthalin,
5-Acetylamino-4-hydroxy-3-[(2'-methylphenyl)azo]-2,7-naphthalin-disulfonsäure,
2-(2',4'-Dimethylphenylazo)-6-(4"-sulfophenylazo)-1,3-dihydroxybenzol,

Als Chinonfarbstoffe können beispielsweise genannt werden:
1,4-Bis-(2',3'-dihydroxypropyl)amino-anthrachinon,
1-Methylamino-4-(2'-hydroxyethyl)amino-anthrachinon,
2-(2'-Aminoethyl)amino-anthrachinon,
2-Brom-4,8-diamino-6-(3'-trimethylammonium)-phenylamino-1,5-naphthochinon,
1-(2'-Sulfo-4'-methyl-phenyl)-amino-4-hydroxy-anthrachinon,
1,4-Diamino-anthrachinon,
1-Amino-2-sulfo-4-cyclohexylamino-anthrachinon,
1-Methylamino-4-aminopropylamino-anthrachinon,
1-Aminopropylamino-anthrachinon,
1,4-Diamino-2-methoxy-anthrachinon
1,4-Bis(2-Hydroxyethyl)amino-5,8-dihydroxy-anthrachinon.

Als Triphenylmethanfarbstoffe können beispielsweise genannt werden:
4',4",4"'-Triamino-3-methyl-triphenylcarboniumchlorid,
Bis-(4,4-Diethylaminophenyl)-4'-ethylamino-naphthyl-carboniumchlorid, Bis-(4,4-Dimethylaminophen)-4'-phenylamino-naphthyl-carbonsiumchlorid, Basic Blue 26, CI 44045
und 4,4-Bis-(N-Ethyl-3-sulfobenzyl)-amino-2"-sulfofuchsonium

Als saure Farbstoffe können beispielsweise genannt werden:
1-(4'-Sulfonphenylazo)-2-hydroxy-6-sulfo-naphthalin Cl 15985,
1-(2'Hydroxy-4'sulfo-6'nitro)-naphthylazo-2-hydroxynaphthalin Cl 15700,
2,4-Dinitro-1-naphthol-7-sulfonsäure-Dinatriumsalz (Acid Yellow 1; Cl 10 316);
2-(2'-Chinolyl)-1H-indene-1,3(2H)-dion-monodisulfon-säure-Dinatriumsalz (Acid Yellow 3; CI 47 005);
4,5-Dihydro-5-oxo-1-(4'-sulfophenyl)-4-[(4"-sulfo-phenyl)azo]-1H-pyrazol-3-carbonsäure-Trinatriumsalz (Acid Yellow 23; Cl 19 140);
3',6'-Dihydroxyspiro [isobenzofuran-1(3H), 9'(9H)-xanthen]-3-on (Acid Yellow 73; Cl 45 350:1);
5-[2',4'-Dinitrophenyl)amino]-2-(phenylamino)-benzol-sulfonsäure-Natriumsalz (Acid Orange 3; CI 10 385);
4-[(2',4'-Dihydroxyphenyl)azo]-benzolsulfonsäure-Natrium-salz (Acid Orange 6; CI 14 270);
4-[2'-Hydroxy-1'-naphthyl)azo]-benzolsulfonsäure-Natriumsalz (Acid Orange 7; CI 15 510);
4-[[3'-[(2",4"-Dimethylphenyl)azo]-2',4'-dihydroxyphenyl]azo]-benzolsulfonsäure-Natriumsalz (Acid Orange 24;CI 20 170);
4-Hydroxy-3-[(4'-sulfo-1'-naphthyl)azo]-1-naphthalin-sulfonsäure-Dinatriumsalz (Acid Red 14; CI 14 720);
7-Hydroxy-8-[(4'-sulfo-1'-naphthyl)azo]-1,3-naphthalin-disulfonsäure-Trinatriumsalz (Acid Red 18; CI 16 255);
3-Hydroxy-4-[(4'-sulfo-1'-naphthyl)azo]-2,7-naphthalin-disulfonsäure-Trinatriumsalz (Acid Red 27; CI 16 185);
5-Amino-4-hydroxy-3-phenylazo-2,7-naphtalindisulfon-säure-Dinatriumsalz (Acid Red 33; CI 17 200);
5-(Acetylamino)-4-hydroxy-3-[(2'-methylphenyl)azo]-2,7-naphthalindisulfonsäure-Dinatriumsalz (Acid Red 35; CI 18 065);
3',6'-Dihydroxy-2',4',5',7'-tetraiodospiro-[isobenzo-furan-1(3H), 9'(9H)-xanthen]-3-on-Dinatriumsalz (Acid Red 51; Cl 45 430);
3,6-Bis-(diethylamino)-9-(2',4'-disulfophenyl)-xanthyliumhydroxid-Natriumslz (Acid Red 52; Cl 45 100);
7-Hydroxy-8-[[4'-(phenylazo) phenyl]azo]-1,3-naphthalindisulfonsäure-Dinatriumsalz (Acid Red 73; Cl 27 290);
2',4',5',7'-Tetrabromo-3',6'-dihydroxyspiro[isobenzo-furan-1 (3H),9'(9H)-xanthen]-3-on-Dinatriumsalz (Acid Red 87; Cl 45 380);
2',4',5',7'-Tetrabromo-4,5,6,7-tetrachloro-3',6'-dihydroxy-spiro[isobenzofuran-1(3H),9'(9H)-xanthen]-3-on-Dinatriumsalz (Acid Red 92; Cl 45 410);
3',6'-Dihydroxy-4',5'-dijodospiro[isobenzofuran-1 (3H),9'(9H)-xanthen]-3-on-Dinatriumsalz (Acid Red 95; Cl 45 425);
Acid Red 195; Acid Blue 9 (Cl 42 090);
2,2'-[(9,10-Dihydro-9,10-dioxo-1,4-anthracendiyl)-diimino]-bis-(5-methyl-benzolsulfonsäue)-Dinatriumsalz (Acid Green 25; Cl 61 570);
N-[4-[[4'-(Dimethylamino)phenyl]-(2"-hydroxy-3",6"-disulfo-1"-naphthyl)-methylen]-2,5-cyclohexadien-1-yliden]-N-methylmethanaminiumhydroxid (Acid Green 50; CI 44 090);
N-[4-[[4'-Diethylamino)phenyl]-(2",4"-disulfophenyl)-methylen]-2,5-cyclohexadien-1-yliden]-N-ethylethan- aminiumhydroxid-Natriumsalz (Acid Blue 1; CI 42 045);
N-[4-[[4'-Diethylamino)phenyl]-(5"-hydroxy-2",4"-disulfo-phenyl)-methylen]-2,5-caclohexadien-1-yliden]-N-ethyl-ethanaminiumhydroxid-Calciumsalz (Acid Blue 3; CI 42 051);
1-Amino-4-(cyclohexy)amino)-9,10-dihydro-9,10-dioxo-2-anthracensulfonsäure-Natriumsalz (Acid Blue 62; Cl 62 045);
2-(1',3'-Dihydro-3'-oxo-5'-sulfo-2'H-indol-2'-yliden)-2,3-dihydro-3-oxo-1H-indol-5-sulfonsäure-Dinatriumsalz (Acid Blue 74; Cl 73 015);
9-(2'-Carboxyphenyl)-3-[(2"-methylphenyl)amino)-6-[(2"'-methyl-4"'-sulfophenyl)amino)]-xanthylium-hydroxid-Natriumsalz (Acid Violet 9; Cl 45 190);
2-[(9',10'-Dihydro-4'-hydroxy-9',10'-dioxo-1'-anthracenyl)-amino]-5-methylbenzolsulfonsäure-Natriumsalz (Acid Violet 43; CI 60 730);
3,3'-[Sulfonyl-bis(2-nitro-4,1-phenylen)imino]-bis-[6-phenylamino)-benzoldinatriumsulfonat] (Acid Brown 13; Cl 10 410);
4-Amino-5-hydroxy-3-[(4'-nitrophenyl)azo]-6-(phenylazo)-2,7-naphthalindisulfonsäure-Dinatriumsalz (Acid Black 1; Cl 20 470);
3-Hydroxy-4-[(2'-hydroxy-1'-naphthyl)azo]-7-nitro-1-naphthalinsulfonsäure-Natriumsalz (Acid Black 52; CI 15 711);
3-[(2,4-Dimethyl-5-sulfophenyl)azo]-4-hydroxy-1-naphthalin-sulfonsäure (Poncean SX, CI 14700).

Als basische Farbstoffe können beispielsweise genannt werden:
Bis-(4,4-Dimethylaminophen)-4'-phenylamino-naphthyl-carbonsiumchlorid,
Basic Blue 26, CI 44045
N-[4-[[4'-(Diethylamino)phenyl]-[4"-(ethylamino)-1"-naphthyl]-methylen]-2,5-cyclohexadien-1-yliden]-N-ethyl-ethanammoniumchlorid (Basic Blue 7; CI 42 595);
4-[(4'-Aminophenyl)-(4'-imino-2',5'-cyclohexadien-1'-yliden)-methyl]-2-methyl-aminobenzol-Hydrochlorid (Basic Violet 14; Cl 42 510);
4-(Acetylamino)-5-hydroxy-6-[[7'-sulfo-4'-[(4"-Sulfo-phenyl)azo]-1'-naphthyl]azo]-1,7-naphthalin-disulfonsäure-Tetranatriumsalz (Brilliant Black 1; CI 28 440);
[8-(p-Aminophenyl)azo]-7-hydroxy-2-naphthyl]-tri-methylammoniumchlorid (Basic Brown 16; CI 12 250);
[8-[4'-Amino-2'-nitrophenyl)azo]-7-hydroxy-2-naphthyl]-trimethylammoniumchlorid (Basic Brown 17; CI 12 251);
7-Hydroxy-8-[(2'-methoxyphenyl)azo]-N,N,N-trimethyl-2-naphthylammoniumchlorid (Basic Red 76; Cl 12 245);
3-[(4'-Amino-6'-bromo-5',8'-dihydro-1'-hydroxy-8'-imino-5'-oxo-2'-naphthyl)amino]-N,N,N-trimethylammonium-chlorid (Basic Blue 99; Cl 56 059).
4-(3'-Trimethylammoniumphenylazo)-N-phenyl-3-methyl-pyrazolon-5 (Basic Yellow 57, CI 12719).

Die Mengen des auf die Haut zu applizierenden Peptids orientiert sich an der Menge und Art des entsprechenden Haarbehandlungsmittel. Das zu verwendende kurzkettige Peptid liegt hierbei in einer wässrigen Lösung vor.
Eine gebrauchsfertige wässrige Lösung kann zwischen 0,01 bis 100 mg/ml Peptid, insbesondere zwischen 0,1 bis 50 mg/ml Peptid, ganz besonders zwischen 0,25 und 40 mg/ml, vorzugsweise zwischen 1,0 und 25 mg/ml Peptid enthalten.

Die wässrige Lösung kann zusätzlich Hilfsstoffe enthalten, insbesondere Antioxidationsmittel (zum Beispiel Tocopherolderivate), Komplexbildner (zum Beispiel Ethylendiamintetraessigsäure EDTA, Diethylentriaminpentaessigsäure DTPA, Nitrilotriessigsäure NTA, Hydroxyethylendiamintriessigsäure HEDTA), Puffer (zum Beispiel citrate buffer citrate-phosphate bufferphosphate buffer), Konservierungsmittel oder antimikrobiell wirkende Agenzien (wie zum Beispiel (Parahydroxybenzoesäure-Ester, Benzylalkohol, Butyl-, Propyl-, Ethyl- und Methylparabene, Natriumhydroxymethylaminoacetat, Methylisothiazolinon, Phenoxyethanol, Quaternium-15), Parfüme, Feuchtigkeitsgeber (wie zum Beispiel Dimethiconesilicon, Lanolin und Lanolinalkohole, Aminosäuren, Panthenol, Sorbitol, Glycerin, Propylenglycol), Viskositätsmodifizierer (wie zum Beispiel Methylcellulose, Xanthan gum, Hydroxyethylcellulose, Methylhydroxyethylcellulose, Polyvinylpyrrolidon, Acrylic-copolymer, Carbomere).

Die Applikation bzw. Formulierung der kurzkettigen Peptide kann auf verschiedene Weise erfolgen, wobei dünnflüssige, viskose, cremige bis pastöse Formulierungen in Betracht kommen. So kann eine Zusammensetzung mit einem Gehalt an kurzkettigem Peptid als Wasser, als Lotion, als Gel, als Paste oder in Form einer Creme vorliegen.

Für den Fall, dass die Haut nicht nur vor Haarbehandlungsmitteln geschützt werden, sondern zusätzlich eine Pflege erhalten soll, können die kurzkettigen Peptide mit einem Pflegemittel verbunden werden. Indsofern umfasst die vorliegende Erfindung auch die nicht medizinische Verwendung einer Zubereitung mit einem Gehalt an kurzkettigen Peptiden zum gleichzeitigen Schutz der Haut vor Haarbehandlungsmitteln und zusätzlich einer Pflege der Haut.

Diese zweifache Wirkung kann dadurch erreicht werden, das mindestens ein pflegender Wirkstoff nach an sich bekannten chemischen Methoden an ein kurzkettiges Peptidmolekül gekoppelt wird.

Hierzu kommen beispielsweise in Frage: Proteinhydrolysate (z.B. aus Weizen), Amidoamine, Feuchtigkeitsgeber wie z.B. Laktate (zum Beispiel Cetyllaktat), Vitamine oder Provitamine bzw. Vitaminvorläufer wie beispielsweise Panthenol und Derivate hiervon, Biotin, Tocopherole, Zucker wie zum Beispiel Polysaccharide, Oligosaccharide, Glucose, Fructose oder Inulin, organischchemische UV-Filter wobei alle bekannten UVA-, UVB- und UVA/UVB-Filtersubstanzen, einzeln oder in Kombination miteinander, in Betracht kommen, wie zum Beispiel die Derivate des Dibenzoylmethans (beispielsweise Parsol 1789 von Givaudan/Roure, INCI-Bezeichung: Butyl Methoxydibenzoylmethane), Benzylidencampher oder Derivate davon, insbesondere Methylbenzylidencampher (beispielsweise 3-Benzylidencampher, 3-(4-Methylenbenzyliden)-dl-campher), Derivate und Ester der Zimtsäure, insbesondere Derivate und Ester der Methoxyzimtsäure (beispielsweise 4-Methoxyzimtsäureoctylester oder 4-Methoxyzimtsäureisopentylester), Derivate und Ester der Benzoesäure, insbesondere der 4-Aminobenzoesäuren, Polyhydroxybenzoesäuren (beispielsweise Polyhydroxybenzoesäuremethylester oder Polyhydroxybenzoesäurepropylester), Ester der Salicylsäure (beispielsweise Salicylsäure(2-ethylhexyl)ester oder Salicylsäure(4-isopropylbenzyl)ester), Sulfonsäuren, Benzophenone und deren Derivate, beispielsweise die Sulfonsäurederivate der Benzophenone (beispielsweise 2-Hydroxy-4-methoxybenzophenon-5-sulfonsäure) als UVB/UVA-Filter oder der Benzimidazole (beispielsweise 2-Phenylbenzimidazol-5-sulfonsäure) sowie deren Salze, Dibenzoylmethane oder geeignete Polypeptide, insbesondere Sauerstoffradikalfänger, beispielsweise die bekannten Mn-, Fe- oder Zn-Superoxiddismutasen, sowie Tocopherole und Vitamine (beispielsweise Ascorbinsäure).

Die genannten pflegenden Wirkstoffe können in einer Gesamtmenge zwischen 0,001 bis 30,0 Gew%, insbesondere zwischen 0,01 bis 25,0 Gew.%, ganz besonders zwischen 0,1 bis 15 Gew.%, vorzugsweise zwischen 0,5 und 10,0 Gew%, bezogen auf die Menge des zu verwendenden kosmetischen Mittels, enthalten sein.

Legenden zu den Figuren:
Figur 1
   Schweinehautpräparate nach Behandlung mit einer Farbstofflösung gemäß Beispiel 2. Die Färbung erfolgte gemäß Beispiel 4. Das linke Präparat wurde vor der Färbung mit einer Peptidlösung gemäß Beispiel 3 vorbehandelt, beim rechten Präparat wurde diese Vorbehandlung unterlassen.

Die nachfolgenden Beispiele sollen die vorliegende Erfindung näher beschreiben.

### Beispiel 1: Herstellung von Schweinehaut Präparaten

Frische Teile von Schweinehaut (mitteleuropäisches Hausschwein), wurden mit einem herkömmlichen milden Shampoo gründlich gereinigt, um sie von Kontaminationen durch die Entnahmprozedur oder durch Handberührungen zu säubern. Von diesen Hautproben wurden Teile von ca, 5,0 x 5,0 cm herausgeschnitten und jeweils in eine Petri-Schale gelegt

### Beispiel 2: Herstellung einer Farbstofflösung

Es wurde eine Lösung folgender Zusammensetzung hergestellt:

| Rohstoff | Menge (Gew.%) |
|---|---|
| Natrosol 250 HHR¹ | 1,0 |
| Nip Nip² | 0,2 |
| Ethanol (96 %) | 5,0 |
| Plantaren 2000 UP³ | 5,0 |
| VE-Wasser | 88,8 |
| | 100 |

| | |
|---|---|
| ¹ Hydroxyethylcellulose ² Methyl parabene 0.14% + Propyl paraben 0.06% ³ Decyl Glucoside (Cognis) | |

Natrosol 250 HHR wurde mit ungefähr einem Drittel des Wassers unter Rühren (Magnetrührer) auf ca. 55°C erhitzt, bis die Lösung dickflüssig wurde.
Ethanol, Plantaren 2000 UP und Nip Nip wurden zusammen mit 0,4 Gew.% Farbstoff (Basic Violet 14) mit dem zweiten Drittel des Wassers vermischt und anschließend zum Kochen gebracht. Die heiße Farbstoff-Lösung wurde unter Rühren (Magnetrührer) in die Natrosollösung gegeben. Mit dem restlichen Wasser wurde das Farbstoffgefäß nachgespült und zu der hergestellten Mischung hinzugegeben. Unter langsamen Rühren (Magnetrührer) wurde im kalten Wasserbad auf Raumtemperatur abgekühlt.
Der pH-Wert kann bei ungefähr 30°C mit Natronlauge oder Zitronensäure auf pH 6,0 eingestellt, wobei Schwankungen im Bereich von pH 0,9 toleriert werden können.

### Beispiel 3: Behandlung von Hautproben mit einem kurzkettigen Peptid

Es wurden je 10 ml einer wässrigen Lösung aus 5,0 mg/ml eines Peptids der Sequenz LITASFTQSLPRKSG hergestellt.
Auf je eine aus Beispiel 1 gewonnene Hautprobe wurde mit einer Pasteur-Pipette jeweils 10 ml dieser Peptid-Lösung aufgetragen. Anschließend wurde diese Lösung bis zur Trockne auf der Hautprobe belassen, wobei innerhalb von ungefähr 10 Minuten unter Zuhilfenahme eines Haartrockners ein vollständiges Trocknen erreicht wurde.
Nach 30 Minuten wurden die Hautproben für 2 Minuten mit Leitungswasser gespült, in eine neue Petri-Schale gegeben und mit einem Haartrockner erneut getrocknet.

### Beispiel 4: Färbung von Hautproben

Mit der gemäß Beispiel 2 hergestellten Farbstofflösung wurden sowohl gemäß Beispiel 3 behandelte Hautproben als auch nicht mit dem kurzkettigen Peptid behandelte Hautproben (Kontrolle) mit einem Pinsel flächendeckend eingestrichen. Nach einer Einwirkzeit von 30 Minuten wurden die Hautpräparate unter Leitungswasser und durch Reiben mit dem Fingern für 2 Minuten gespült.

Anschließend wurden sie in eine Petri-Schale gegeben und mit einem Haatrockner getrocknet.

Als Ergebnis konnte festgestellt werden, dass die mit den beiden Peptiden behandelten Hautproben gegenüber der unbehandelten Hautproben eine sehr geringe Anfärbung aufwiesen.

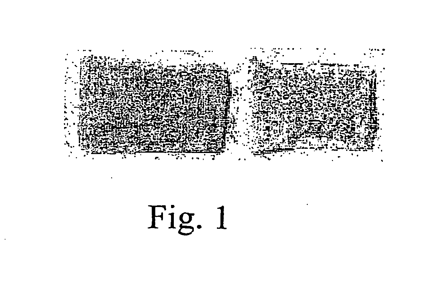

## Patentansprüche

1. Nicht medizinische Verwendung von kurzkettigen Peptiden mit einer Kettenlänge zwischen 2 und 30 Aminosäuren zum Schutz der Haut vor Haarbehandlungsmitteln.

2. Verwendung nach Anspruch 1, **dadurch gekennzeichnet, dass** der Schutz der Haut vor Haarbehandlungsmitteln solche Mittel umfasst, die das Haar färben, tönen, verformen, härten, konditionieren, weichmachen, reparieren oder stylen können.

3. Verwendung nach Ansprüchen 1 und 2, **dadurch gekennzeichnet, dass** der Schutz der Haut vor Haarbehandlungsmitteln solche Mittel umfasst, die das Haar färben oder tönen können.

4. Verwendung nach Ansprüchen 1 bis 3, **dadurch gekennzeichnet, dass** die kurzkettigen Peptide eine Kettenlänge zwischen 6 und 15 Aminosäuren, insbesondere zwischen 6 und 12 Aminosäuren, aufweisen.

5. Verwendung nach Ansprüchen 1 bis 4 zum gleichzeitigen Schutz der Haut vor Haarbehandlungsmitteln und zusätzlich einer Pflege der Haut.

## Claims

1. Non-medical use of short-chain peptides having a chain length between 2 and 30 amino acids to protect the skin against hair-treatment compositions.

2. Use according to Claim 1, **characterized in that** the protection of the skin against hair-treatment compositions includes those compositions which can dye, tint, shape, harden, condition, soften, repair or style hair.

3. Use according to Claims 1 and 2, **characterized in that** the protection of the skin against hair-treatment compositions includes those compositions which can colour or tint the hair.

4. Use according to Claims 1 to 3, **characterized in that** the short-chain peptides have a chain length between 6 and 15 amino acids, in particular between 6 and 12 amino acids.

5. Use according to Claims 1 to 4 for the simultaneous protection of the skin against hair-treatment compositions and additionally care of the skin.

## Revendications

1. Utilisation non médicinale de peptides à courte chaîne, présentant une longueur de chaîne comprise entre 2 et 30 acides aminés pour la protection de la peau vis-à-vis de produits de traitement capillaire.

2. Utilisation selon la revendication 1, **caractérisée en ce que** la protection de la peau vis-à-vis de produits de traitement capillaire englobe les produits qui peuvent teindre, nuancer, mettre en forme, fixer, conditionner, assouplir, réparer ou coiffer les cheveux.

3. Utilisation selon les revendications 1 et 2, **caractérisée en ce que** la protection de la peau vis-à-vis de produits de traitement capillaire englobe les produits qui peuvent teindre ou nuancer les cheveux.

4. Utilisation selon les revendications 1 à 3, **caractérisée en ce que** les peptides à courte chaîne présentent une longueur de chaîne comprise entre 6 et 15 acides aminés, en particulier entre 6 et 12 acides aminés.

5. Utilisation selon les revendications 1 à 4, pour la protection de la peau vis-à-vis de produits de traitement capillaire et simultanément, de surcroît,pour un soin de la peau.
